(19) Europäisches Patentamt
European Patent Office
Office européen des brevets

(11) **EP 1 990 009 B1**

(12) **EUROPEAN PATENT SPECIFICATION**

(45) Date of publication and mention
of the grant of the patent:
**15.04.2015 Bulletin 2015/16**

(51) Int Cl.:
*A61B 8/00* (2006.01)      *A61B 8/08* (2006.01)
*G01S 7/52* (2006.01)

(21) Application number: **06835168.3**

(22) Date of filing: **25.12.2006**

(86) International application number:
**PCT/JP2006/325721**

(87) International publication number:
**WO 2007/097108 (30.08.2007 Gazette 2007/35)**

(54) **ULTRASONIC DIAGNOSTIC EQUIPMENT**

ULTRASCHALL-DIAGNOSEGERÄT

EQUIPEMENT DE DIAGNOSTIC ULTRASONORE

(84) Designated Contracting States:
**DE FR GB IT NL**

(30) Priority: **22.02.2006 JP 2006044649**

(43) Date of publication of application:
**12.11.2008 Bulletin 2008/46**

(73) Proprietor: **Hitachi Medical Corporation**
**Tokyo 101-0021 (JP)**

(72) Inventors:
• **YOSHIKAWA, Hideki**
**Kokubunji-shi Tokyo 185-0014 (JP)**
• **AZUMA, Takashi**
**Kokubunji-shi Tokyo 185-0014 (JP)**
• **HAYASHI, Tatsuya**
**Tokyo 101-0021 (JP)**

(74) Representative: **Strehl Schübel-Hopf & Partner**
**Maximilianstrasse 54**
**80538 München (DE)**

(56) References cited:
WO-A1-2005/020821      DE-A1-102006 027 992
JP-A- 08 266 541      JP-A- 10 075 949
JP-A- 2006 325 686      US-A- 5 495 846

• ISHIHARA K ET AL: "High-speed digital subtraction echography: principle and preliminary application to arteriosclerosis, arrhythmia and blood flow visualization", 19901204; 19901204 - 19901207, 4 December 1990 (1990-12-04), pages 1473-1476, XP010010006,
• YOSHIKAWA H ET AL: "Time-averaged high contrast ultrasound imaging with motion correction", ULTRASONICS SYMPOSIUM, 2005 IEEE ROTTERDAM, THE NETHERLANDS 18-21 SEPT. 2005, PISCATAWAY, NJ, USA,IEEE, vol. 1, 18 September 2005 (2005-09-18), pages 524-527, XP010898794, DOI: DOI:10.1109/ULTSYM. 2005.1602906 ISBN: 978-0-7803-9382-0
• YOSHIKAWA H; AZUMA T; SASAKI K; KAWABATA K; UMEMURA S: "Visualization of tumor's vascularity for HIFU treatment with tissue motion correction", THERAPEUTIC ULTRASOUND: 5TH INTERNATIONAL SYMPOSIUM ON THERAPEUTIC ULTRASOUND, 27 October 2005 (2005-10-27), - 29 October 2005 (2005-10-29), pages 71-75, XP002639836, Boston, MA,USA ISSN: 0094-243X
• YOSHIKAWA H; AZUMA T; KAWABATA K; TANIGUCHI Y; UMEMURA S: "Three-dimensional tracking method of tissue motion with biplane images", 25TH SYMPOSIUM ON ULTRASONIC ELECTRONICS (USE2004), 27 October 2004 (2004-10-27), - 29 October 2004 (2004-10-29), pages 4561-4566, XP002639837, Sapporo, Japan ISSN: 0021-4922

**Description**

Technical Field

The present invention relates to an apparatus for displaying a high contrast ultrasonic image with reduced speckle noise.

Background Art

An ultrasonic diagnostic apparatus that can noninvasively pickup an image of an affected area in real time has been widely used as a monitoring tool for initial diagnosis, fetal diagnosis, and treatment at a clinic. In recent years, portable ultrasonic diagnostic apparatuses have been developed, which facilitates emergency treatments with the apparatus carried to the patient at any place in or out of hospitals. In addition to the portable size of the apparatus, the easy operation of the ultrasonic diagnostic apparatus promotes the scalable network between a patient's house and a hospital, thereby the achievement of home medical care can be expected in which the patient himself/herself transfers an image of him/her to the hospital while operating the apparatus for a remote diagnosis.

An ultrasonic image can be advantageously picked up in a short period of time and displayed in real time, but disadvantageously has a weak signal strength which indicates a tissue structure relative to a noise level (low S/N ratio), which often makes diagnosis of the image difficult. Thus, in order to promote the accuracy in diagnosis and the information sharing between a doctor and a patient, a technology for displaying an image having more objective information in a manner easy to understand is expected.

An ultrasonic image has mottles called speckles, which is one of the factors that makes the diagnosis of an image difficult. Spatial compound sonography is a method for reducing the speckles and displaying a high quality ultrasonic image. The speckles on a two dimensional ultrasonic image (B-mode image) are the spatially standing signals which are generated when the signals reflected from minute scatterers interfere with each other, the scatterers being dispersed in an object under inspection, and have a spatial frequency which is lower than that of a signal indicating a structure of the object under inspection. As a result, when a surface for image pickup is moved in a slice direction by a distance which is equal to the size of a speckle, the speckles randomly change on the image, while the structure of the object under inspection does not significantly change. If such images acquired at slightly different positions in the slice direction are added to each other, the signals from the structure of the object under inspection is enhanced at every addition, but the signals from the randomly changing speckles become smoothed. As a result, an S/N ratio between a speckle and the structure is improved, thereby a high contrast image having an enhanced S/N ratio is constructed.

So far, a number of technologies for adding a plurality of images and extracting specific information therefrom have been reported, and a few of them will be explained below.

Patent Document 1 describes a technology in which a plurality of blood flow images (CFM: Color Flow Mapping) are stored in a memory, and an operator selectively removes frames which are used in an adding process among the displayed images, so that the remained images are added to construct an image.

Patent Document 2 relates to a technology in which signals are received in an efficient and stable manner from a contrast medium after a small amount of the contrast medium is given, and the distribution of blood vessels can be clearly imaged. An imaging of blood vessel information requires a crush of the contrast medium which is flowing in the blood using ultrasonic waves, and a construction of an image by receiving strong nonlinear signals which are generated at the crush. Because strong ultrasonic waves are necessary in crushing a contrast medium, there is a problem that quite different S/Ns are generated between the area where the transmission signals are focused and the other areas, and the image by a contrast medium has non-uniformity in the direction of its depth. To address the problem, in the technology described in Japanese Patent Application Laid-Open No. 2002-209898, the area where the transmission signals are focused are changed in the depth direction at a number of stages to construct a plurality of images which is called as a group of multi-shot images, on which an adding process is performed. In addition, a specific area of each frame of the group of multi-shot images is weighted before the adding process.

Patent Document 1: Japanese Patent No. 3447150
Patent Document 2: Japanese Patent Laid-Open No. 2002-209898

In constructing an image by performing an adding/subtracting process on a plurality of images to enhance specific information of the images, and displaying the image in real time, problems occur such as a shift of the position of a subject, an increased time for processes, and an increased memory.

The technology described in Patent Document 1, which was made without consideration of any position shift of an object under inspection, is hardly applied to an object under inspection which is movable. In addition, an operator selects an image to be used in an adding process, which makes it impossible to display the image in real time. The technology described in Patent Document 2 has another problem that, in constructing and displaying one image in an adding process, every frame to be added has to be stored in a memory once, and the number of frames in the memory has to be larger than that required in the adding process.

Further, US 5 495 846 discloses an ultrasonic diagnostic imaging apparatus which applies a weighting to newly acquired image frame data and old image frame data before both are added to obtain an updated version of the old image frame data which thus represents a moving average of the acquired image frames. The invention has the features recited in the precharacterising first part of claim 1 in common with this prior art. In the prior art, the weighting depends on the difference between the old image frame data, i.e. the previously acquired moving average, and the newly acquired image frame data.

K. Ishihara et al.: "High-speed digital subtraction echography: Principle and preliminary application to arteriosclerosis, arrhythmia and blood flow visualization", 1990 Ultrasonics Symposium, pages 1473 to 1476 discloses an ultra-sonic diagnostic imaging apparatus which obtains the difference between consecutive images, to visualize motion.

Disclosure of the Invention

One object of the present invention is to provide an ultrasonic diagnostic apparatus which displays a high contrast ultrasonic image with reduced speckles. This object is solved by an apparatus as set forth in claim 1.

Embodiments of the present invention extract information which changes over time. In embodiments of the present invention, a weighting is applied to a cumulative added image, and an operator controls the amount of information which changes over time and is extracted from the images to be used.

In order to achieve the above described objects, in the ultrasonic diagnostic apparatus of an embodiment of the present invention, a motion vector (information of stop-motion) of an object under inspection which is generated between a captured B-mode image and another B-mode image captured just before the image is detected, and based on the detected motion vector, an image transformation process is performed while an image addition process is performed by multiplying a weight factor, so that an enhanced image of an edge structure of the object under inspection is displayed.

Now, the structures of typical examples of an ultrasonic diagnostic apparatus will be listed below.

(1) An ultrasonic diagnostic apparatus which includes: an ultrasonic transducer for transmitting/receiving an ultrasonic wave to be radiated to an object under inspection; an ultrasonic image constructing unit for using a signal of the received ultrasonic wave and constructing a B-mode image which shows a transverse image of the object under inspection; an image memory #1 for storing a B-mode image $f_{n-1}$ of the next to the last frame; a measurement region setting section for defining on an image $f_{n-1}$ at least one region at which a motion vector of the object under inspection is detected; a motion detector for detecting a motion vector of the object under inspection which is generated between the image $f_{n-1}$ and the image $f_n$; an added image generating section for generating a cumulative added image which is constructed using images from an image $f_1$ to an image $f_{n-1}$; an image transforming (correcting) section for transforming (correcting) the cumulative added image which is constructed using images from an image $f_1$ to an image $f_{n-1}$ based on the detected result of the motion vector; a weight parameter unit for multiplying at least one of an acquired image and the cumulative added image by a weight factor; an accumulation unit for performing an adding process on the cumulative image and the acquired image; an image memory #2 for storing a cumulative added image constructed by the accumulation unit to be used in an adding process performed on an image $f_{n+1}$ which will be captured next; and a displaying section for displaying the cumulative added image. Hereinafter, the phrase "to transform an added image" includes a correction of the added image based on a motion vector.

(2) The ultrasonic diagnostic apparatus according to the above description (1) is characterized in that the detection of a motion vector is carried out between the acquired B-mode image $f_n$ and the B-mode image $f_{n-1}$ of the next to the last frame, and the adding process is performed by using the acquired B-mode image $f_n$ as a reference image and transforming the cumulative added image $\sum_{i=1}^{n-1} f_i$ which is constructed by the B-mode images of the previous frames.

(3) The ultrasonic diagnostic apparatus according to the above description (1) is characterized in that a new added image is constructed by performing an adding process in which the acquired image and the cumulative added image is multiplied by a weight factor.

(4) The ultrasonic diagnostic apparatus according to the above description (1) is characterized in that the weight factor multiplied to the acquired image and the cumulative added images is any value depending on an operator.

(5) The ultrasonic diagnostic apparatus according to the above description (1) is characterized in that the weight factor multiplied to the acquired image and the cumulative added images is automatically adjusted by a correlation value which is calculated in the detection of the motion vector, and for example, in a case with a larger motion of the object under inspection, the weight factor is set to be 0 for an acquired image.

According to the present invention, a high contrast image in which an edge structure of tissues is enhanced and the speckle noise is reduced is achieved, thereby an ultrasonic image having high visibility can be displayed.

Brief Description of the Drawings

Figure 1 is a block diagram showing a structure of an ultrasonic diagnostic apparatus of Example 1;
Figure 2 is a flowchart illustrating processes from a capturing of a B-mode image to a displaying of a cumulative added image in the ultrasonic diagnostic apparatus of Example 1;
Figure 3 is a diagram illustrating an approach for detecting a motion vector in the ultrasonic diagnostic apparatus of Example 1;
Figure 4 is a diagram illustrating an image process for constructing a new cumulative added image using an acquired image and a cumulative added image of the previous frames in the ultrasonic diagnostic apparatus of Example 1;
Figure 5 is a flowchart illustrating a process for performing a detection of a motion vector at a high speed in the ultrasonic diagnostic apparatus of Example 1;
Figure 6 is a diagram illustrating an approach for detecting a motion vector in the ultrasonic diagnostic apparatus of Example 1;
Figure 7 is a diagram showing a weight factor of each frame for constructing a cumulative added image in the ultrasonic diagnostic apparatus of Example 1;
Figure 8 is a diagram showing an example of a transducer or ultrasonic diagnostic device having a dial for adjusting a weight factor ($\alpha,\beta$) in the ultrasonic diagnostic apparatus of Example 1;
Figure 9 is a diagram showing an example of a transducer or ultrasonic diagnostic device having a combination of dial and button which adjusts and also refreshes a weight factor ($\alpha,\beta$) in the ultrasonic diagnostic apparatus of Example 1;
Figure 10 is a diagram showing an example of a bi-plane display in which a B-mode image and an added image are displayed side by side in the ultrasonic diagnostic apparatus of Example 1;
Figure 11 is a block diagram showing a structure of an ultrasonic diagnostic apparatus of Example 2;
Figure 12 is a block diagram showing a structure of an ultrasonic diagnostic apparatus of Example 3;
Figure 13 is a diagram illustrating a unit image which is constructed with five images in the ultrasonic diagnostic apparatus of Example 3; and
Figure 14 is a diagram showing weight factors for five unit images and one image in the ultrasonic diagnostic apparatus of Example 3.

Best Mode for Carrying Out the Invention

Now, examples of the present invention will be explained in detail below with reference to the drawings.

Example 1

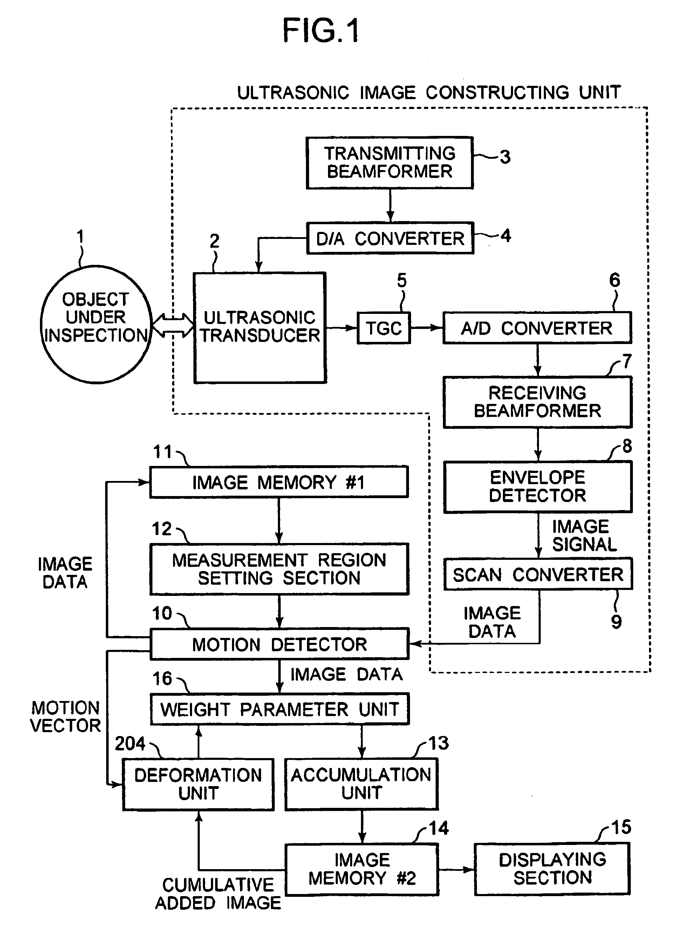

Figure 1 is a block diagram showing a structure of an ultrasonic diagnostic apparatus.
In an ultrasonic diagnostic apparatus of the present example, a two dimensional ultrasonic image (hereinafter, referred to as a B-mode image) is constructed by transmitting/receiving ultrasonic waves to and from an object under inspection, and then a region on the B-mode image in which a motion vector of the object under inspection is detected is defined, so that a motion vector generated between a currently captured B-mode image and a B-mode image of the next to the last frame is detected in every measurement region, and based on the motion vector, a cumulative added image is transformed (corrected), which is multiplied by a weight factor and added to the captured B-mode image, thereby a new cumulative added image is constructed and displayed on a displaying section in real time.
First, with reference to the block diagram of Figure 1, description will be made below to the structure of the apparatus starting from a construction of a B-mode image in an ultrasonic image constructing unit up to a display of an added image after an image adding process with position correction.
An ultrasonic transducer (hereinafter, referred to as a transducer) 2 is configured with a plurality of piezo-electric devices which are arranged in parallel to each other. An analog signal is transmitted from a transmitting beamformer 3 to each of the piezo-electric devices through a D/A converter 4, which cases ultrasonic waves to be radiated to an object under inspection 1. The ultrasonic waves transmitted from each of the piezo-electric devices are electronically delayed by the transmitting beamformer 3 to be focused at a predefined depth. The signal of the transmitted waves is reflected within the object under inspection 1 to be received at each of the piezo-electric devices in the transducer again. The reflected echo received by each of the piezo-electric devices includes attenuated components which are variable depending on the depth where the transmitted waves reach and are corrected at a TGC (Time Gain Control) section 5, and then is converted to a digital signal at an A/D converter 6 to be transmitted to a receiving beamformer 7.

At the receiving beamformer 7, a result of addition is output after a multiplication by a delay time corresponding to a distance from a focal point to each of the piezo-electric devices. The focused ultrasonic waves are two dimensionally scanned to obtain a two dimensional distribution of the reflected echo from the object under inspection 1. The receiving beamformer 7 outputs an RF signal having a real part and an imaginary part which are sent to an envelope detector 8 and a measurement region setting section 11. The signal sent to the envelope detector 8 is converted into a video signal, and is compensated between the scan lines therein by a scan converter 9, so as to construct a B-mode image, that is two dimensional image data.

The constructed B-mode image is sent to a motion detector 10. At this point of time, in an image memory #1 11, a B-mode image of one frame before the B-mode image which is captured in the motion detector 10 is stored. When the constructed B-mode image is the image of the first frame, the image is not processed at the motion detector 10 but passes there through, to be input into the image memory #1 11. On the stored B-mode image in the image memory #1 11, a measurement region having the most appropriate size at which a motion vector is detected is defined by a measurement region setting section 12 depending on the size of the structure of the subject under inspection. After the definition of the measurement region, the B-mode image is sent to the motion detector 10. At the motion detector 10, the B-mode image from the measurement region setting section 12 and the B-mode image from the scan converter 9 are used to detect a motion vector in the measurement region. An approach for detecting a motion vector is a cross correlation method or a least square method. In a deformation unit 13, based on the motion vector detected in the motion detector 10, a cumulative added image which is read out from an image memory #2 14 is transformed. In a weight parameter unit 16, the acquired image and the cumulative added image are multiplied by a weight factor, and in the accumulation unit 13, the acquired image and the cumulative added image are added. The cumulative added image constructed in the accumulation unit 13 is once stored in an image memory #2 14 and then displayed on the displaying section 15. The cumulative added image stored in an image memory #2 14 is sent to the accumulation unit 13 via the weight parameter unit 16 when a next adding process is performed.

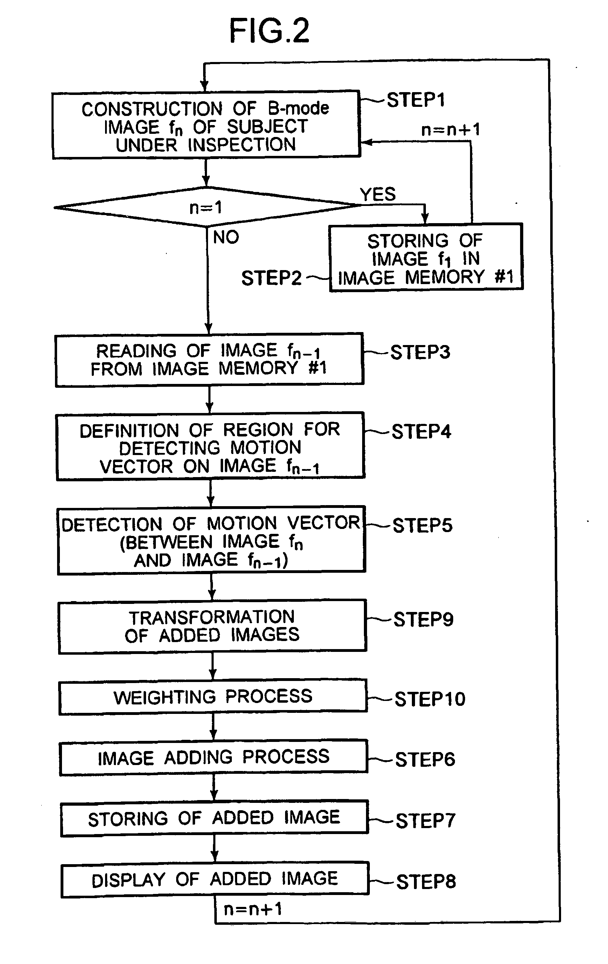

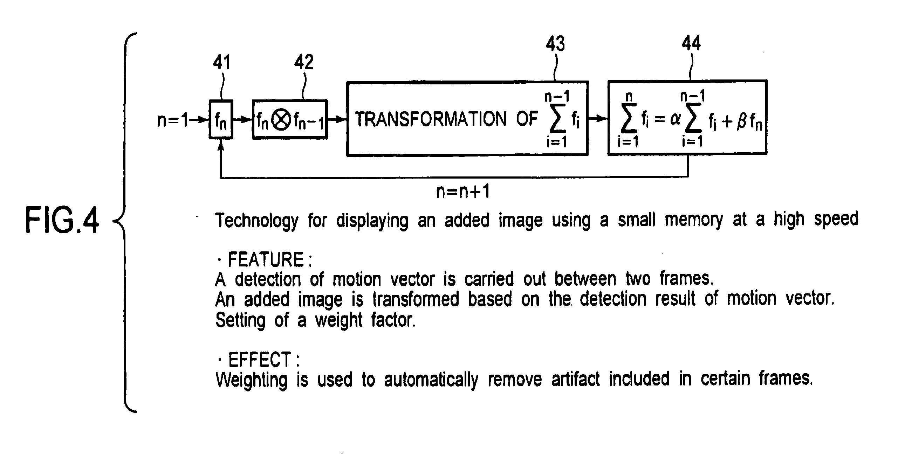

Next, in accordance with the flowchart of Figure 2, steps from obtaining of a B-mode image to displaying a cumulative added image will be explained in detail below. Figure 4 is a block diagram showing a flow of the image processes.

First, at Step 1, a B-mode image $f_n$ is constructed. When the constructed B-mode image $f_n$ is the first image (n = 1), the image $f_n$ is stored in the image memory #1 11 (Step 2), and a next B-mode image is captured (Figure 4-41). Simultaneously with the sending of the image $f_n$ (n > 1) to the motion detector, the image $f_{n-1}$ of the next to the last frame which is stored in the image memory #1 11 is read in (Step 3) and a region on the image at which a motion vector is detected is defined (Step 4). Subsequently, using the image $f_n$ and the image $f_{n-1}$, a motion vector generated between the images is detected for each defined measurement region (Step 5, Figure 4-42). Next, a cumulative added image $\sum_{i=1}^{n-1} f_i$ of the images of the first to n-1st frames is read in from the image memory #2 14, which is subjected to a transformation process based on the motion vector detected at Step 5 (Step 9, Figure 4-43). Then, the cumulative added image after the transformation process is subjected to a weighting process in which the cumulative added image is multiplied by a certain weight factor (Step 10). The cumulative added image $\sum_{i=1}^{n-1} f_i$ subjected to the transformation process and the weighting process is then subjected to an adding process at the accumulation unit 13 using the image $f_n$ as a reference (Step 6, Figure 4-44). The new cumulative added image $\sum_{i=1}^{n} f_i$ constructed as described above is stored in the image memory #2 14 (Step 7), to be displayed on the displaying section 15 (Step 8).

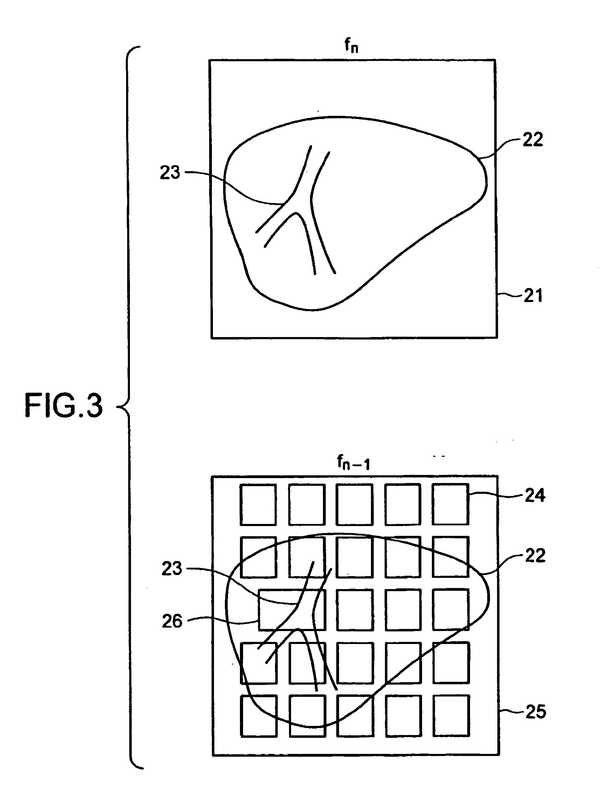

An approach for defining a measurement region at Step 4 will be explained below by way of Figure 3.

In the present invention, a plurality of measurement regions 24 are defined in a B-mode image ($f_{n-1}$) 25, and one measurement region which best matches with each of the measurement region 24 is searched out from an acquired B-mode image ($f_n$) 21 using a cross correlation method or a least square method. Any motion within each one measurement region is reckoned as a rigid body motion without transformation, and individual motion vectors obtained in each of the measurement regions are combined, so that the entire motion of the object under inspection with transformation is detected. The measurement regions are defined in the B-mode image ($f_{n-1}$) 25 so as to make the regions uniform, the regions being used in an adding process using a weight factor, which will be explained later. A weight factor is set to be large relative to an added image. On the contrary, if measurement regions are defined in the acquired B-

mode image ($f_n$) 21, the regions extracted from an added image include an added region and a non-added region having speckles. Thus, an adding process using such regions causes the speckles to round up by a weight factor, which produces artifact.

The signal components used in a detection can be generally classified in two types: low frequency components for example at edge structures or boundaries between tissues of an object under inspection; and speckles of high frequency components due to the interferences between ultrasonic waves which are scattered by the minute scatterers dispersed in the object under inspection. In the present invention, these two types are not distinguished from each other, and the measurement regions are defined all over an image to calculate motions. When a B-mode image is used to detect a motion between images, the speckle of high frequency components not only enhances the accuracy in detection of a motion vector, but also enables the detection of a motion at a substantial site of tissues that has no characteristic structure. A measurement region should have a size larger than a speckle which is the minimum element of a B-mode image, and is defined to have a size about twice to three times that of the speckle. As for the abdominal tissues such as a liver or a kidney, a measurement region of about $5 \times 5$ mm$^2$ is defined.

For the definition of a measurement region, alternatively, an approach for defining measurement regions having different sizes for different tissue parts may be used. A measurement region should have a smaller size to obtain a higher detection resolution of a motion vector, but in this case, the number of the regions is naturally increased, which increases the processing time required for the detection. Therefore, a measurement region which includes a characteristic structure such as an edge of tissues is defined to have a larger size in accordance with the spatial frequency in the region than the other regions. For example, in Figure 3, when a measurement region is defined in an image 25 including liver tissue 22 and vascular structure 23 in the liver tissue, a measurement region 26 including a vascular structure is defined to have a larger size than that of peripheral measurement regions 24 that have no characteristic structure. A measurement region including a tissue boundary or a measurement region with a large transformation has a correlation value lower than those of peripheral areas, which makes the detection of a motion vector of the tissue difficult. Thus, another approach may be used to redefine a measurement region having a low correlation value to have a size which is no more than twice that of a speckle.

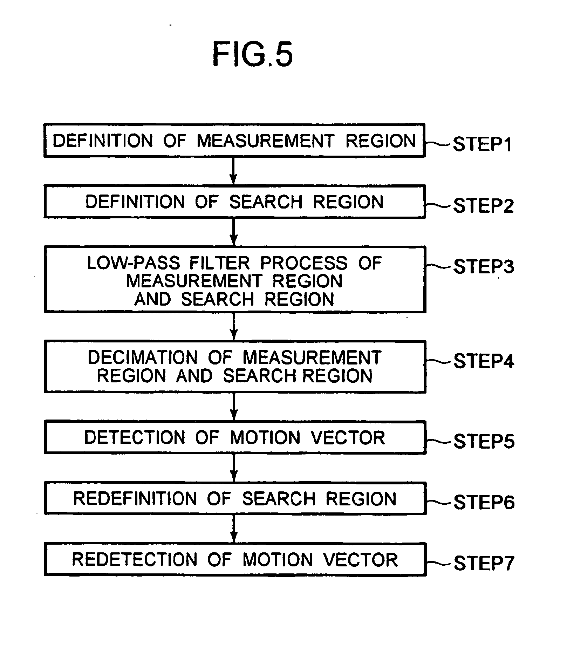

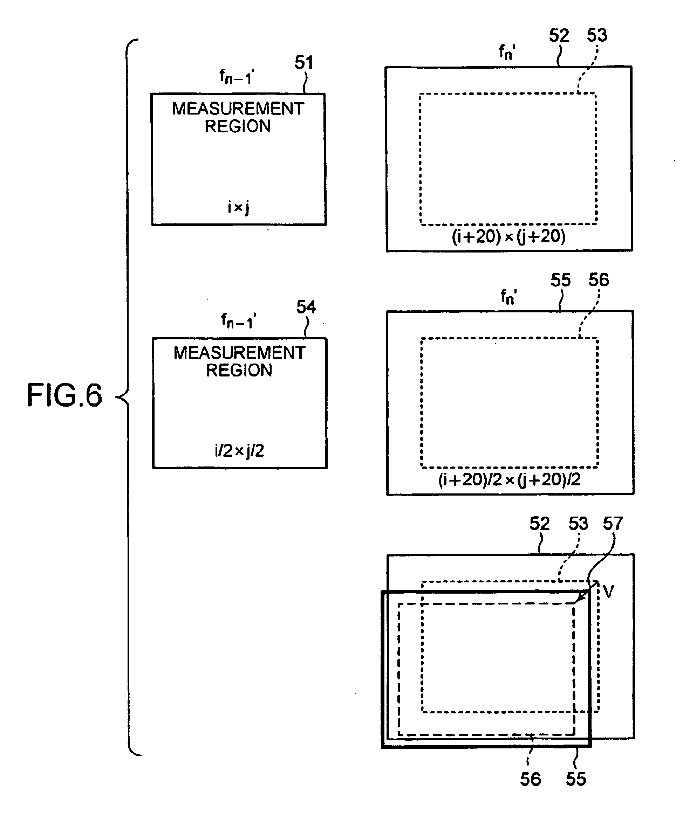

Next, a specific approach for a motion vector detection at Step 5 and an image adding process at Step 6 will be explained below by way of Figures 5 and 6. The detection of a motion vector is carried out using a captured image $f_n$ and an image $f_{n-1}$ of the next to the last frame. The process flow of the motion vector detection will be explained below by way of the flowchart shown in Figure 5. First, a measurement region in which a motion vector is detected is defined in the image $f_{n-1}$ using the approach described above (Step 1). Actually a plurality of measurement regions are defined all over the screen, but in Figure 5, among the regions, only one measurement region 51 is shown as the one having a size of (i,j). Next, a search region $f_n'$ 52 for searching for a region which best matches most with the measurement region $f_{n-1}'$ 51 is defined in the image $f_n'$ (Step 2). The search region $f_n'$ 52 is defined to be as small as possible in consideration of the speed at which an object under inspection moves and a frame rate. In the case of a liver which is affected by breathing at a frame rate of 20-30 frames/sec, a detection of a motion vector can be carried out when a region is defined to have a size of (i+20, j+20) which is larger than the measurement region $f_{n-1}'$ 51 by 10 pixels from each side of the measurement region $f_{n-1}'$ 51. Next, a low-pass filter is applied to the measurement region $f_{n-1}'$ 51 and the search region $f_n'$ 52 (Step 3), so that a decimation process is performed for every pixel to eliminate picture elements (Step 4). The low-pass filter process prevents aliasing between picture elements due to a subsequent decimation process, which can reduce the process time required for a region search to about one fourth. A specific method of the decimation process will be explained below by way of Figure 6. Within the search region $f_n'$ 55 after being subjected to the decimation process, the measurement region $f_{n-1}'$ 54 also after the decimation process is scanned pixel by pixel so as to search for the position having the minimum correlation value c which is defined by the following equation (1) or (2), so that a motion vector V57 is detected (Step 5).

$$c = \sum_{k=1}^{20} \sum_{l=1}^{20} [\{f_n'(k,l) - f_{n-1}'(\mathrm{k},\mathrm{l})\}^2] \quad (\text{equation1})$$

$$c = \sum_{k=1}^{20} \sum_{l=1}^{20} [|f_n'(k,l) - f_{n-1}'(\mathrm{k},\mathrm{l})|] \quad (\text{equation2})$$

The symbol || in the equation 2 represents an absolute value. The detection of a motion vector using the image after a decimation process may include a detection error of $\pm$ 1 pixel. To eliminate the error, a measurement region 56 is redefined from the image $f_n$ by moving the measurement region 51 from the original position by the motion vector V57, and the search region 55 is redefined from the image $f_{n-1}$ to be larger than the measurement region 56 by 1-2 pixels from each side of the measurement region 56 (Step 6). The redefined measurement region 56 and the search region 55 are used to detect again a motion vector V2 using the similar approach as that at Step 5 (Step 7). Through the above described processes, the motion vector to be corrected in an adding process eventually has a value of $((2 \times V) + V2)$ with a use of the motion vector V57.

An adding process transforms a cumulative added image which is constructed with images of from the first frame to the next to the last frame and adds it to a captured image. The adding process is performed with an acquired image as a reference, thereby the tissues are positioned in the same relationship with the cumulative added image $\sum_{i=1}^{n-1} f_i$ and the image $f_{n-1}$. After the detection of the motion vector, the cumulative added image of from the first frame to the n-1$^{st}$ frame $\sum_{i=1}^{n-1} f_i$ is read in from the added images storing section 14 to the weight parameter unit 16 to be multiplied by a weight factor, which is input to the accumulation unit 13 to be subjected to a transformation process using the motion vector $((2 \times V) + V2)$ detected in the motion detector. The weighting to the cumulative added image $\sum_{i=1}^{n-1} f_i$ after the transformation process is carried out in a form expressed as the following equation 3 which uses a weight factor $(\alpha,\beta)$, so as to construct a new cumulative added image.

$$\sum_{i=1}^{n} f_i = \alpha \sum_{i=1}^{n-1} f_i + \beta f_n \qquad \text{(equation 3)}$$
$$(\alpha + \beta = 1)$$

When the equation 3 is expanded to indicate the factors for each of the frames which construct the cumulative added image, the following equation 4 can be obtained.

$$\sum_{i=1}^{n} f_i = \alpha^{n-1} f_1 + \alpha^{n-2}\beta f_2 + \Lambda + \alpha\ \beta f_{n-1} + \beta f_n \qquad \text{(equation 4)}$$

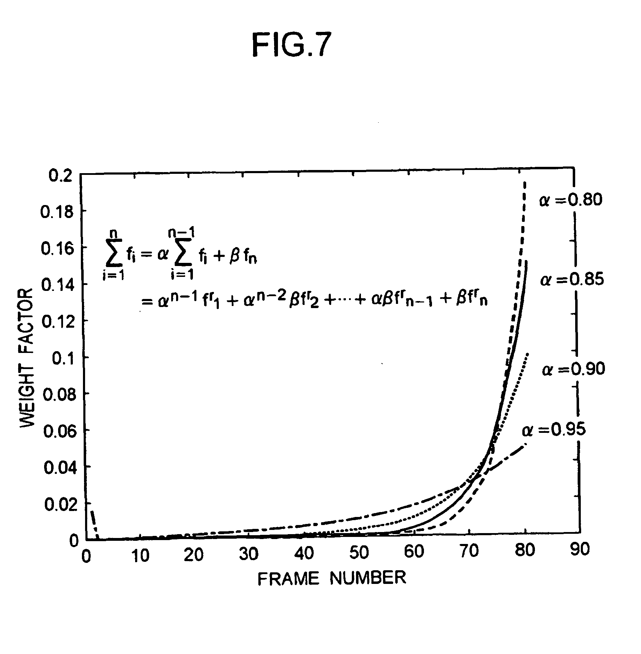

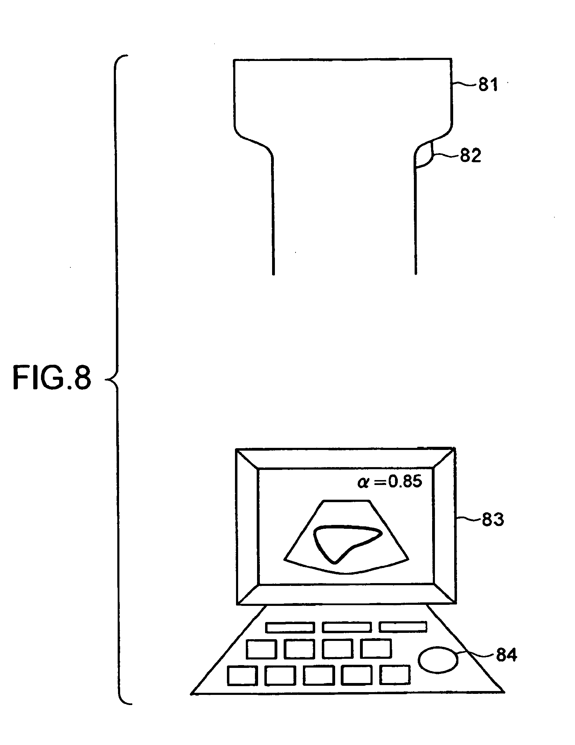

The weight factor of each frame which can be obtained by the equation 4 is an important parameter to determine the effect of addition. Figure 7 shows the weight factor of each frame, where $(\alpha,\beta)$ = (0.95, 0.05), (0.90, 0.1), (0.85, 1.5), (0.8, 0.2), and n = 80. With a larger $\alpha$ value (e.g., $\alpha$ = 0.80), the weight factor rapidly increases at the 70$^{th}$ to 80$^{th}$ frames, resulting in a modest effect in edge enhancement or speckle removal. However, as to the first to 70$^{th}$ frames, due to the extremely low weight factor, the residual detection error which generated in the past is reduced. To the contrary, with a smaller $\alpha$ value (e.g., $\alpha$ = 0.95), the graph extends more horizontally than the case with a larger $\alpha$ value, and the weight factor of each frame is similar to each other. Many frames affect the cumulative added image, which provide a larger adding effect but also cause the detection error in the past in any one frame to be remained in the resulting cumulative added image. The most appropriate level of edge enhancement or speckle removal depends on an operator or a subject under inspection. Therefore, the value $(\alpha,\beta)$ is set to have an initial value (0.85, 0.15) so that an operator can change the value $(\alpha,\beta)$ as needed. The initial value can be changed to any by an operator. In order to change the $\alpha$ value in operation, as shown in Figure 8, a dial 82 attached to a transducer 81 or a dial 84 mounted to a diagnostic device 83 may be rotated, for example. In changing, the current $\alpha$ value is displayed on the screen of the diagnostic device. The above described weight factor is intended to adjust the effect in addition by its multiplication to both a cumulative added images and an acquired image, and is not limited to the manner expressed in the equation 3, thereby various factor types may be used such as the number of weight factors and multipliers of weight factors.

There can be several method for controlling a weight for a cumulative added image and controlling any residual error. In a first method, an operator manually adjusts a dial. When an operator moves a transducer by a considerable distance to search for a region of interest, the $\alpha$ value is set to be low so as to display an image similar to an ordinary B-mode image, so that the $\alpha$ value is increased for inspecting the region of interest. Also, when the operator wants to delete any residual error, the $\alpha$ value may be temporarily set to be low so as to reduce the artifact due to the detection error. In a second method, the $\alpha$ value is automatically controlled in accordance with the size of a detected motion vector. When the equation 1 and the equation 2 have the value c which is larger than a predetermined threshold, the $\alpha$ value is automatically decreased and the residual error is reduced. In other words, the second method is the automated first method. In a third method, a refresh button (delete button) is provided so that a diagnostic device or transducer causes the added images to be deleted from a memory to perform an adding process again from the first frame. The button allows an operator to eliminate any residual error to 0 as needed. A press down of the refresh button causes the value

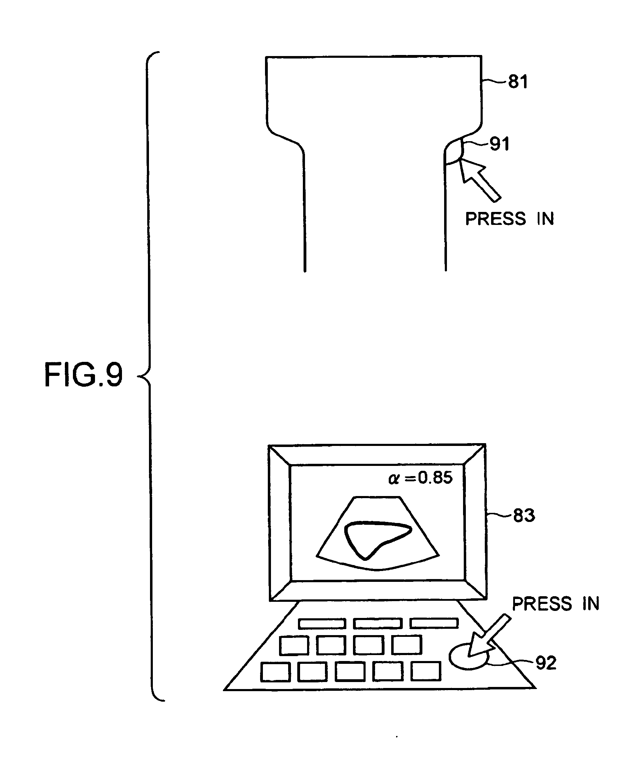

$\alpha$ = 0 to be input in an adding process. The refresh button may be provided separately from a dial which changes the $\alpha$ value shown in Figure 8, but in terms of complexity and convenience of the apparatus, as shown in Figure 9, the apparatus can be operated most easily when an image is refreshed by a press-in of a dial 91 or a dial 92 provided on the transducer 81 or the diagnostic device 83 for changing the $\alpha$ value. When an adding process is performed on the images from the first frame by the refresh button, speckles suddenly become evident, which may make the observation difficult for some operators. Thus, the $\alpha$ value by the refresh button can be set as needed by an operator.

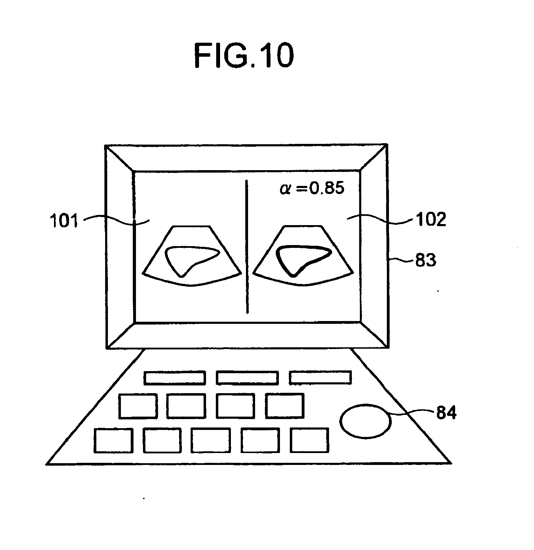

The added image may be displayed all over a screen, but as shown in Figure 10, a B-mode image 101 and an added image 102 may be displayed as bi-plane images side by side. Also, in the explanation of the above example, a B-mode image is used as an ultrasonic image, but the ultrasonic waves diagnostic apparatus of the example 1 may be applied to any image including a THI (Tissue Harmonic Image) in which high frequency components are selectively imaged and a CFM (Color Flow Mapping) image in which a blood flow is imaged.


Example 2

A second example of the present invention will be explained below. The second example is characterized in that, during the motion detection process and the adding process shown in the first example, an added image is constructed with the certain number of frames in another memory, and when an operator uses the refreshing function, the certain number of added frames can be displayed as a cumulative added image.

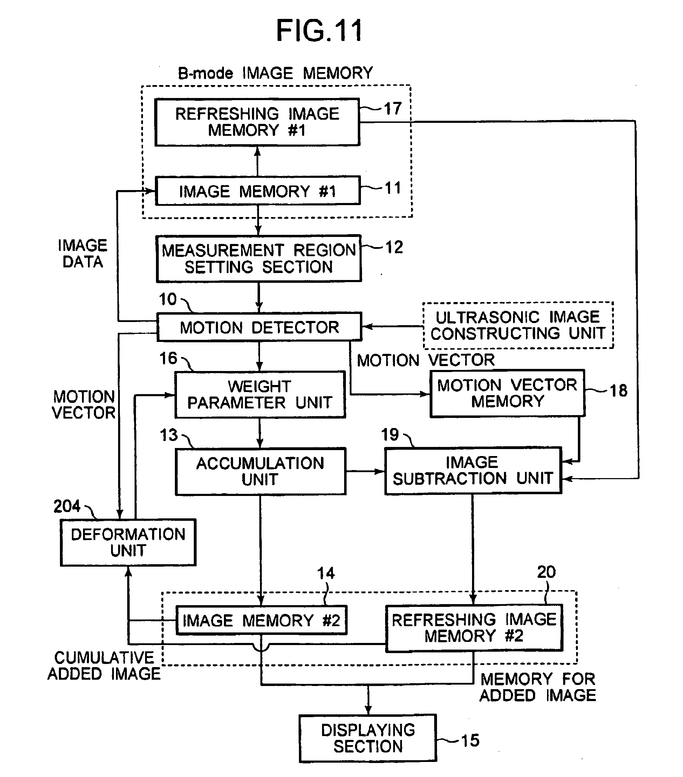

The structure of the apparatus will be explained below by way of the block diagram shown in Figure 11. The processes for constructing added images which include a construction of a B-mode image, a motion vector detection process, and an image addition process are similar to those in the above example 1. In the second example, in addition to the structure of the apparatus of the first example shown in Figure 1, the apparatus further includes a refreshing image memory #1 17, a motion vector memory 18, an image subtraction unit 19, and a refreshing image memory #2 20.

The number of frames for constructing a refreshing added image depends on the number of memories that can be loaded, but the present example will be explained below with five frames.

As in the case of the example 1, an image of the frame which is captured just before the current image is stored in the image memory #1 11 for a detection of a motion vector, but in the example 2, four images in total of two to five frames before the current image are stored in the refreshing image memory #1 17.

After a capture of an image is started, and as the motion vector detection process and the image addition process progress and a cumulative added image $\sum\limits_{i=1}^{5} f_i$ of the five frames is constructed at the accumulation unit 13, the cumulative added image passes through the image subtraction unit 19, and is stored in the refreshing image memory #2 20. The result of a motion vector detection of each image in the motion detector 10 is stored in the motion vector memory 18. Also, as the image data stored in a B-mode image memory at this stage, an image $f_5$ is stored in the image memory #1 11 and the images $f_4$, $f_3$, $f_2$, and $f_1$ are stored in the refreshing image memory #1 17.

Next, when a new image $f_6$ is captured into the accumulation unit 13 through the motion detector 10, and a cumulative added image $\sum\limits_{i=1}^{6} f_i$ is constructed, the cumulative added image $\sum\limits_{i=1}^{6} f_i$ is stored in the image memory #2 14 and at the same time, is input into the image subtraction unit 19. At this point of time, the image $f_5$ is input from the image memory #1 11 into the refreshing image memory #1 17, and at the same time, the image $f_1$ is output to the image subtraction unit 19. In the image subtraction unit 19, a subtraction process is performed to subtract the image $f_1$ from the added images $\sum\limits_{i=1}^{6} f_i$. Actually, the image $f_1$ itself is not subtracted, but the information of the image $f_1$ of what is done to the image $f_1$ from an adding process of the image $f_1$ to the addition of the image $f_6$ including a transformation process is subtracted. Since the information of misalignment between images is stored in the motion vector memory 18, an addition of such information forms a transformation history of a specified image. Thus, when all of the motion vectors detected in the motion detector 10 in the image $f_2$ to image $f_6$ are added, and the image $f_1$ is transformed based on the adding result, the image $f_1$ is constructed as it is included in the added images $\sum\limits_{i=1}^{6} f_i$. When the transformed image $f_1$ is subtracted from the cumulative added image $\sum\limits_{i=1}^{6} f_i$, an added image $\sum\limits_{i=2}^{6} f_i$ constructed with five frames from the second to sixth frames for refreshing is constructed. The added image $\sum_{i=2}^{6} f_i$ is stored in the refreshing image memory #2 20. Because the image $f_2$ will be subtracted in the next procedure, the motion vector detected between the image $f_1$ and the image $f_2$ is deleted from the motion vector memory 18.

Through the above described steps, an added image of five frames from an acquired image is constantly stored in the refreshing image memory #2 20. And when the refresh function is activated, the refreshing added image is displayed on the displaying section 15 instead of the cumulative added image stored in the image memory #2 14, so that the refreshing added image is stored in the image memory #2 14 as a new cumulative added image.

Example 3

A third example of the present invention will be explained below. The third example is characterized in that, an added image of acquired and cumulative added images is divided in a plurality of unit to construct unit images, and a weight factor is assigned to each unit image, so that an added image which is effective in addition and has reduced residual error is displayed.

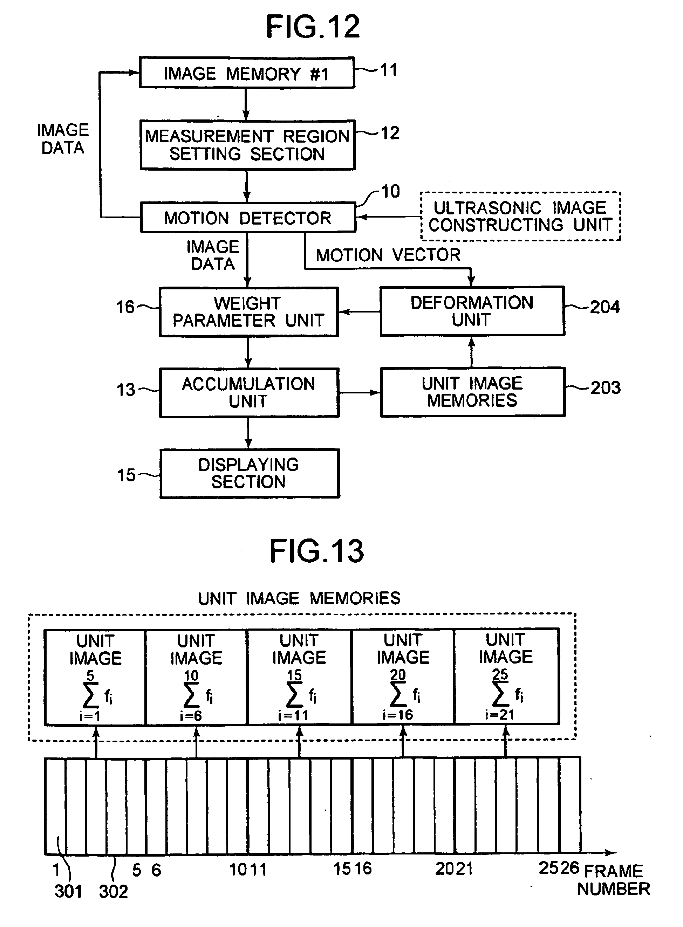

The structure of the apparatus is shown in Figure 12. Now, the processing steps will be explained below on the assumption that the number of frames an operator predetermines to add is 30, and that the number of the images for constructing a unit is five.

The step for detecting a motion vector by using an image $f_n$ input from an ultrasonic image constructing unit and an image $f_{n-1}$ of one frame before the image $f_n$ is similar to those in the above examples 1 and 2. However, in the present example 3, when an added image of five frames is constructed in the accumulation unit 13, the image is stored in the unit image memories 203 as a unit image. That is, for example, when the adding process for a 26th image $f_{26}$ is finished, five unit images $\sum_{i=1}^{5} f_i$ 、 $\sum_{i=6}^{10} f_i$ 、 $\sum_{i=11}^{15} f_i$ 、 $\sum_{i=16}^{20} f_i$ 、 $\sum_{i=21}^{25} f_i$ are stored in total in the unit image memories 203 (Figure 13).

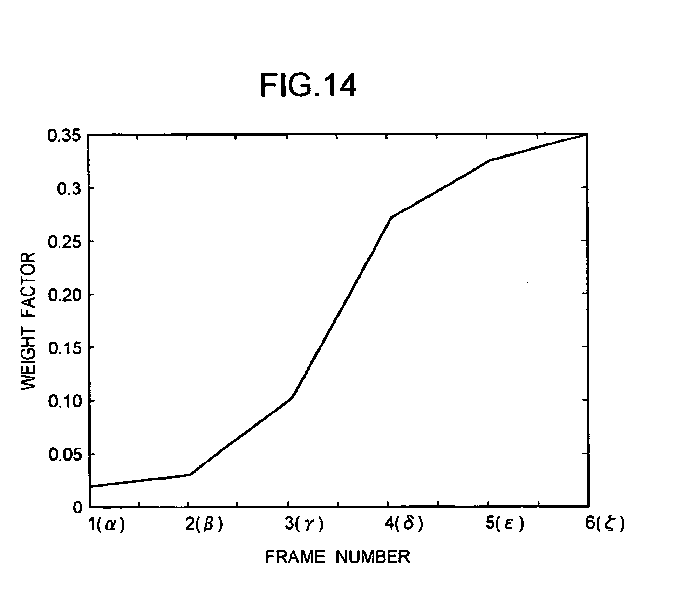

When the image $f_{26}$ is input into the accumulation unit 13, the five unit images stored in the unit image memories 203 are captured in a deformation unit 204, where a transformation process is performed based on a motion vector detected in the motion detector 10. The image subjected to the transformation process is input to the weight parameter unit 16 to be multiplied by a weight factor together with an acquired image $f_{26}$, on which an adding process is performed in an accumulation unit 13 as expressed by the following equation 5. The adding process is performed by multiplying each of the five unit images and the captured image $f_{26}$ by a predetermined weight factor, and for example when the weight factor is set to be (0.02, 0.03, 0.1, 0.27, 0.325, 0.35), the distribution of the weight factors of each unit image relative to the added images can be shown by the graph of Figure 14.

$$\sum_{i=1}^{26} f_i = \alpha \sum_{i=1}^{5} f_i + \beta \sum_{i=6}^{10} f_i + \gamma \sum_{i=11}^{15} f_i + \delta \sum_{i=16}^{20} f_i + \varepsilon \sum_{i=21}^{25} f_i + \zeta f_{26} \quad \text{(equation 5)}$$

$$(\alpha + \beta + \gamma + \delta + \varepsilon + \zeta = 1)$$

When a 31st image $f_{31}$ is captured, a newly constructed unit image $\sum_{i=26}^{30} f_i$ is stored in the unit image memories 203 and also the existed unit image $\sum_{i=1}^{5} f_i$ is deleted, so that a next adding process is performed on a unit image $\sum_{i=6}^{10} f_i$ . Each unit image used in the adding process is subjected to a transformation process in the accumulation unit 13, which is stored again in the unit image memories 203.

As described above, each unit is multiplied by a weight factor, which enables an automatic reduction of residual errors while the adding effect being maintained. And in accordance with the detection result of motion vector, a weight factor for a certain unit can be automatically controlled, for example a smaller weight factor for a unit with a larger detection error.

In the explanation of the above example, the number of the added frames is 30 and the number of images for constructing a unit is five, but as described in the example 1, an adjustment of a weight factor in accordance with the motion size of an object under inspection enables a control of an adding effect and residual error as needed. Moreover, the smaller number of the images for constructing a unit enables a control of a weight factor on a frame basis, which enables a control of an adding effect as needed, and also a removal of a specific frame with large error from an adding process.

**Claims**

1. A diagnostic imaging apparatus, which comprises:

  a transducer (2) for transmitting/receiving an ultrasonic signal at a measurement region of an object;
  an image data generating section (5-9) for generating image data corresponding to a plurality of frames based on each of a plurality of signals from the first to nth frames, which are received by the transducer;
  an image data storing section (11) for storing the image data;
  an added image generating section (13) for generating added image data by adding image data that individually correspond to the frames;
  an added image data storing section (14) for storing the added image data generated by the added image generating section (13) by adding a plurality of two dimensional ultrasonic images from a two dimensional ultrasonic image f1 of the first frame to the two dimensional ultrasonic image fn-1;
  an image processing section (13, 16) for generating display image data; and
  a displaying section (15) for making a display based on the display image data,
  wherein the apparatus comprises:

    a weight parameter unit (16) for multiplying at least one of the added image and the image fn by a weight factor;
    an accumulation unit (13) for performing an adding process on the image fn and the added image after the multiplication by the weight factor, to construct a new added image; and
    an image memory (14) for storing the added image constructed by the accumulation unit (13),
    **characterised in that**
    the image data storing section (11) is adapted to store a two dimensional ultrasonic image fn-1 of an n-1th frame,

  and **in that** the apparatus comprises:

    a measurement region setting section (12) for defining at least one region for detecting a motion vector of the object;
    a motion detector (10) for detecting a motion vector of the object from the image fn-1 and a two dimensional ultrasonic image fn of an nth frame;
    a motion vector memory (18) for storing the detected motion vector;
    an image correcting section (204) for generating corrected image data by reading out the added image data and the motion vector from the added image data storing section (14) and the motion vector memory (18) and correcting the added image data based on the motion vector;
    a further image memory (17) for storing images (f2, f3, ..., fn-i) from the second to (n-i)th frames;
    an image subtraction unit (19) for subtracting a past image from the added image to construct a refreshed added image which is constructed from a certain number of added images; and
    a refreshed image memory (20) for storing the refreshed added image,
    wherein the displaying section (15) is adapted to display the added image or the refreshed added image.

2. The ultrasonic diagnostic apparatus according to claim 1, **characterized in that**
  the motion detector is adapted to detect the motion vector between a two dimensional ultrasonic image of the currently acquired frame and a two dimensional ultrasonic image of one frame before the currently acquired frame,
  the image correcting section is adapted to correct the added image based on the detection result of the motion vector, and
  the accumulation unit is adapted to perform the adding process by multiplying a two dimensional ultrasonic image of the currently acquired frame and the added image by a certain weight factor.

3. The ultrasonic diagnostic apparatus according to claim 1, **characterized in that** the detection of the motion vector is carried out from the two dimensional ultrasonic image of the currently acquired frame and the two dimensional ultrasonic image of one frame before the currently acquired frame and, based on the detected motion vector, the added image which is constructed with two dimensional ultrasonic images from the first frame to the next to the last frame is transformed, and the adding process is performed on the acquired image so as to construct a new added image.

4. The ultrasonic diagnostic apparatus according to claim 1, **characterized in that** a weight factor is provided in the adding process to the currently acquired two dimensional ultrasonic image and the added image, and the adding process is carried out by multiplying both of the images by a certain weight factor.

5. The ultrasonic diagnostic apparatus according to claim 1, **characterized in that** the weight factor is automatically adjusted in accordance with a correlation value which indicates a detection accuracy of the motion vector.

6. The ultrasonic diagnostic apparatus according to claim 1, **characterized in that** it further comprises a dial for changing the weight factor.

7. The ultrasonic diagnostic apparatus according to claim 6, **characterized in that** the displaying section is adapted to display the value of the weight factor changed by the dial.

8. The ultrasonic diagnostic apparatus according to claim 1, **characterized in that** an adding process is carried out on the detected motion vector between the images, so that a transformation history of the image is calculated before a subtraction process to remove information only of a specific image from a cumulative added image.

9. An ultrasonic diagnostic apparatus according to claim 1, **characterized in that** it further comprises:

unit image memories (203) for storing a unit image which is constructed by adding any number of images.

10. The ultrasonic diagnostic apparatus according to at least one of claims 1 and 9, **characterized in that** the measurement region setting section is adapted to define the region on the image fn-1.

11. The ultrasonic diagnostic apparatus according to claim 9, **characterized in that** the unit image memories are adapted to store the plurality of unit images, and the weight parameter unit is adapted to multiply at least one or each of the plurality of unit images by a weight factor.

12. The ultrasonic diagnostic apparatus according to claim 9, **characterized in that** the unit image memories are adapted to store the plurality of unit images, and the image correcting section is adapted to correct each of the plurality of unit images.

13. An ultrasonic diagnostic apparatus according to claim 9, adapted to control the weight factor for a unit image in accordance with the motion vector.

**Patentansprüche**

1. Diagnostische Bildgebungsvorrichtung aufweisend:

einen Übertrager (2) zum Senden bzw. Empfangen eines Ultraschallsignals in einem Messbereich eines Objekts, einen Bilddaten-Erzeugungsabschnitt (5-9) zum Erzeugen von Bilddaten, die mehreren Einzelbildern entsprechen, aufgrund der jeweiligen Signale vom ersten zum n-ten Einzelbild, die vom Übertrager empfangen werden, einen Bilddaten-Speicherabschnitt (11) zum Speichern der Bilddaten, einen Summenbild-Erzeugungsabschnitt (13) zum Erzeugen von Summenbilddaten durch Addieren von Bilddaten, die einzeln den Einzelbildern entsprechen, einen Summenbilddaten-Speicherabschnitt (14) zum Speichern der Summenbilddaten, die vom Summenbild-Erzeugungsabschnitt (13) durch Addieren mehrerer zweidimensionaler Ultraschallbilder von einem zweidimensionalen Ultraschallbild f1 eines ersten Einzelbilds zum zweidimensionalen Ultraschallbild fn-1 erzeugt werden, einen Bildverarbeitungsabschnitt (13, 16) zum Erzeugen von Anzeigebilddaten, und einen Anzeigeabschnitt (15) zum Erzeugen einer Anzeige aufgrund der Anzeigebilddaten, wobei die Vorrichtung aufweist:

eine Gewichtungsparametereinheit (16) zum Multiplizieren des Summenbilds und/oder des Bilds fn mit einem Gewichtungsfaktor,

eine Akkumulationseinheit (13) zum Ausführen eines Additionsprozesses mit dem Bild fn und dem Summenbild nach der Multiplikation mit dem Gewichtungsfaktor, um ein neues Summenbild aufzubauen, und einen Bildspeicher (14) zum Speichern des von der Akkumulationseinheit (13) aufgebauten Summenbilds, **dadurch gekennzeichnet, dass**

der Bilddaten-Speicherabschnitt (11) eingerichtet ist, ein zweidimensionales Ultraschallbild fn-1 eines n-1-ten Einzelbilds zu speichern,

und dass die Vorrichtung aufweist:

einen Messbereichs-Festlegungsabschnitt (12) zum Bestimmen mindestens eines Bereichs zum Erfassen eines Bewegungsvektors des Objekts,
einen Bewegungsdetektor (10) zum Erfassen eines Bewegungsvektors des Objekts aus dem Bild fn-1 und einem zweidimensionalen Ultraschallbild fn des n-ten Einzelbilds,
einen Bewegungsvektorspeicher (18) zum Speichern des erfassten Bewegungsvektors,
einen Bildkorrekturabschnitt (204) zum Erzeugen korrigierter Bilddaten durch Auslesen der Summenbilddaten und des Bewegungsvektors aus dem Summenbilddaten-Speicherabschnitt (14) und dem Bewegungsvektorspeicher (18) und zum Korrigieren der Summenbilddaten aufgrund des Bewegungsvektors,
einen weiteren Bildspeicher (17) zum Speichern von Bildern (f2, f3, ..., fn-i) vom zweiten zum (n-i)-ten Einzelbild,
eine Bildsubtraktionseinheit (19) zum Subtrahieren eines vergangenen Bilds vom Summenbild, um ein aufgefrischtes Summenbild zu erzeugen, das aus einer gewissen Anzahl addierter Bilder aufgebaut ist, und einen Frischbildspeicher (20) zum Speichern des aufgefrischten Summenbilds,
wobei der Anzeigeabschnitt (15) eingerichtet ist, das Summenbild oder das aufgefrischte Summenbild anzuzeigen.

2. Ultraschalldiagnostische Vorrichtung nach Anspruch 1, **dadurch gekennzeichnet, dass**
der Bewegungsdetektor eingerichtet ist, den Bewegungsvektor zwischen einem zweidimensionalen Ultraschallbild des gegenwärtig gewonnenen Einzelbilds und einem zweidimensionalen Ultraschallbild eines Einzelbilds vor dem gegenwärtig gewonnenen zu erfassen,
der Bildkorrekturabschnitt eingerichtet ist, das Summenbild aufgrund des Erfassungsergebnisses für den Bewegungsvektor zu korrigieren, und
die Akkumulationseinheit eingerichtet ist, den Additionsprozess auszuführen, indem ein zweidimensionales Ultraschallbild des gegenwärtig gewonnenen Einzelbilds und das Summenbild mit einem gewissen Gewichtungsfaktor multipliziert werden.

3. Ultraschalldiagnostische Vorrichtung nach Anspruch 1, **dadurch gekennzeichnet, dass** die Erfassung des Bewegungsvektors ausgehend vom zweidimensionalen Ultraschallbild des gegenwärtig gewonnenen Einzelbilds und des zweidimensionalen Ultraschallbilds eines Einzelbilds vor dem gegenwärtig gewonnenen ausgeführt wird und aufgrund des erfassten Bewegungsvektors das Summenbild, das mittels der zweidimensionalen Ultraschallbilder vom ersten zum vorletzten Einzelbild aufgebaut wird, umgewandelt wird und mit dem gewonnenen Bild der Additionsprozess ausgeführt wird, um ein neues Summenbild aufzubauen.

4. Ultraschalldiagnostische Vorrichtung nach Anspruch 1, **dadurch gekennzeichnet, dass** im Additionsprozess für das gegenwärtig gewonnene zweidimensionale Ultraschallbild und das Summenbild ein Gewichtungsfaktor vorgesehen ist und der Additionsprozess durch Multiplizieren beider Bilder mit einem gewissen Gewichtungsfaktor ausgeführt wird.

5. Ultraschalldiagnostische Vorrichtung nach Anspruch 1, **dadurch gekennzeichnet, dass** der Gewichtungsfaktor entsprechend einem Korrelationswert, der eine Erfassungsgenauigkeit für den Bewegungsvektor angibt, automatisch eingestellt wird.

6. Ultraschalldiagnostische Vorrichtung nach Anspruch 1, **dadurch gekennzeichnet, dass** sie einen Wähler zum Ändern des Gewichtungsfaktors aufweist.

7. Ultraschalldiagnostische Vorrichtung nach Anspruch 6, **dadurch gekennzeichnet, dass** der Anzeigeabschnitt eingerichtet ist, den vom Wähler geänderten Wert des Gewichtungsfaktors anzuzeigen.

8. Ultraschalldiagnostische Vorrichtung nach Anspruch 1, **dadurch gekennzeichnet, dass** mit dem erfassten Bewegungsvektor zwischen den Bildern ein Additionsprozess ausgeführt wird, sodass eine Umwandlungsgeschichte des Bilds berechnet wird, bevor ein Subtraktionsprozess stattfindet, um lediglich von einem bestimmten Bild eines kumulativ summierten Bilds Information zu entfernen.

**9.** Ultraschalldiagnostische Vorrichtung nach Anspruch 1, **gekennzeichnet durch**:

Einheitsbildspeicher (203) zum Speichern eines Einheitsbilds, das **durch** Addieren irgendeiner Anzahl an Bildern aufgebaut wird.

**10.** Ultraschalldiagnostische Vorrichtung nach mindestens einem der Ansprüche 1 und 9, **dadurch gekennzeichnet, dass** der Messbereichs-Festlegungsabschnitt eingerichtet ist, den Bereich auf dem Bild fn-1 festzulegen.

**11.** Ultraschalldiagnostische Vorrichtung nach Anspruch 9, **dadurch gekennzeichnet, dass** die Einheitsbildspeicher eingerichtet sind, mehrere Einheitsbilder zu speichern, und die Gewichtungsparametereinheit eingerichtet ist, mindestens eines oder jedes der Einheitsbilder mit einem Gewichtungsfaktor zu multiplizieren.

**12.** Ultraschalldiagnostische Vorrichtung nach Anspruch 9, **dadurch gekennzeichnet, dass** die Einheitsbildspeicher eingerichtet sind, mehrere Einheitsbilder zu speichern, und der Bildkorrekturabschnitt eingerichtet ist, jedes der Einheitsbilder zu korrigieren.

**13.** Ultraschalldiagnostische Vorrichtung nach Anspruch 9, die zur Steuerung des Gewichtungsfaktors für ein Einheitsbild entsprechend dem Bewegungsvektor eingerichtet ist.

**Revendications**

**1.** Appareil de diagnostic par imagerie, lequel comporte :

un transducteur (2) pour transmettre/recevoir un signal ultrasonore au niveau d'une région de mesure d'un objet, une section de génération de données d'images (5-9) pour générer des données d'images correspondant à une pluralité de trames sur la base de chaque signal parmi une pluralité de signaux depuis la première trame jusqu'à la n$^{\text{ème}}$ trame, lesquelles sont reçus par le transducteur, une section de stockage de données d'images (11) pour stocker les données d'images, une section de génération d'images ajoutées (13) pour générer des données d'images ajoutées en ajoutant des données d'images qui correspondent individuellement aux trames, une section de stockage de données d'images ajoutées (14) pour stocker les données d'images ajoutées générées par la section de génération d'images ajoutées (13) en ajoutant une pluralité d'images ultrasonores bidimensionnelles depuis une image ultrasonore bidimensionnelle f1 de la première trame jusqu'à l'image ultrasonore bidimensionnelle fn-1, une section de traitement d'images (13, 16) pour générer des données d'images d'affichage, et une section d'affichage (15) pour effectuer un affichage sur la base des données d'images d'affichage, dans lequel l'appareil comporte :

une unité de paramètres de pondération (16) pour multiplier au moins l'une de l'image ajoutée et de l'image fn par un facteur de pondération, une unité d'accumulation (13) pour exécuter un processus d'ajout sur l'image fn et l'image ajoutée après la multiplication par le facteur de pondération, pour construire une nouvelle image ajoutée, et une mémoire d'images (14) pour stocker l'image ajoutée construite par l'unité d'accumulation (13), **caractérisé en ce que** la section de stockage de données d'images (11) est adaptée pour stocker une image ultrasonore bidimensionnelle fn-1 d'une (n-1)$^{\text{ème}}$ trame,

et **en ce que** l'appareil comporte :

une section d'établissement de région de mesure (12) pour définir au moins une région pour détecter un vecteur de mouvement de l'objet, un détecteur de mouvement (10) pour détecter un vecteur de mouvement de l'objet à partir de l'image fn-1 et une image ultrasonore bidimensionnelle fn d'une n$^{\text{ème}}$ trame,

une mémoire de vecteurs de mouvement (18) pour stocker le vecteur de mouvement détecté,
une section de correction d'images (204) pour générer des données d'images corrigées en extrayant les données d'images ajoutées et le vecteur de mouvement de la section de mémorisation de données d'images ajoutées (14) et de la mémoire de vecteurs de mouvement (18) et en corrigeant les données d'images ajoutées sur la base du vecteur de mouvement,
une mémoire d'images supplémentaire (17) pour mémoriser des images (f2, f3,..., fn-i) depuis la deuxième jusqu'à la (n-i)$^{ème}$ trame,
une unité de soustraction d'images (19) pour soustraire une image précédente de l'image ajoutée afin de construire une image ajoutée rafraîchie qui est construite à partir d'un nombre particulier d'images ajoutées, et
une mémoire d'images rafraîchies (20) pour mémoriser l'image ajoutée rafraîchie,
dans lequel la section d'affichage (15) est adaptée pour afficher l'image ajoutée ou l'image ajoutée rafraîchie.

2. Appareil de diagnostic à ultrasons selon la revendication 1 **caractérisé en ce que**
le détecteur de mouvement est adapté pour détecter le vecteur de mouvement entre une image ultrasonore bidimensionnelle de la trame actuellement acquise et une image ultrasonore bidimensionnelle d'une trame avant la trame actuellement acquise,
la section de correction d'images est adaptée pour corriger l'image ajoutée sur la base du résultat de détection du vecteur de mouvement, et
l'unité d'accumulation est adaptée pour exécuter le processus d'ajout en multipliant une image ultrasonore bidimensionnelle de la trame actuellement acquise et l'image ajoutée par un vecteur de pondération particulier.

3. Appareil de diagnostic à ultrasons selon la revendication 1, **caractérisé en ce que** la détection du vecteur de mouvement est exécutée à partir de l'image à ultrasons bidimensionnelle de la trame actuellement acquise et de l'image à ultrasons bidimensionnelle d'une trame avant la trame actuellement acquise et, sur la base du vecteur de mouvement détecté, l'image ajoutée qui est construite avec deux images à ultrasons bidimensionnelles de la première trame à l'avant-dernière trame est transformée, et le processus d'ajout est exécuté sur l'image acquise de manière à construire une nouvelle image ajoutée.

4. Appareil de diagnostic à ultrasons selon la revendication 1, **caractérisé en ce que** un facteur de pondération est fourni dans le processus d'ajout à l'image ultrasonore bidimensionnelle actuellement acquise et l'image ajoutée, et le processus d'ajout est exécuté en multipliant les deux images par un facteur de pondération particulier.

5. Appareil de diagnostic à ultrasons selon la revendication 1, **caractérisé en ce que** le facteur de pondération est automatiquement ajusté conformément à une valeur de corrélation qui indique une précision de détection du vecteur de mouvement.

6. Appareil de diagnostic à ultrasons selon la revendication 1, **caractérisé en ce que** il comporte en outre un cadran pour changer le facteur de pondération.

7. Appareil de diagnostic à ultrasons selon la revendication 6, **caractérisé en ce que** la section d'affichage est adaptée pour afficher la valeur du facteur de pondération changé par le cadran.

8. Appareil de diagnostic à ultrasons selon la revendication 1, **caractérisé en ce que** un processus d'ajout est exécuté sur le vecteur de mouvement détecté entre les images, de sorte qu'un historique de transformation de l'image est calculé avant un processus de soustraction pour éliminer des informations uniquement d'une image spécifique à partir d'une image ajoutée cumulative.

9. Appareil de diagnostic à ultrasons selon la revendication 1, **caractérisé en ce que** elle comporte en outre :

des mémoires d'images unitaires (203) pour stocker une image unitaire qui est construite en ajoutant un nombre quelconque d'images.

10. Appareil de diagnostic à ultrasons selon au moins l'une des revendications 1 et 9, **caractérisé en ce que** la section d'établissement de région de mesure est adaptée pour définir la région de l'image fn-1.

11. Appareil de diagnostic à ultrasons selon la revendication 9, **caractérisé en ce que** les mémoires d'images unitaires sont adaptées pour stocker la pluralité d'images unitaires, et l'unité de paramètres de pondération est adaptée pour

**14**

multiplier au moins l'une ou chacune de la pluralité d'images unitaires par un facteur de pondération.

12. Appareil de diagnostic à ultrasons selon la revendication 9, **caractérisé en ce que** les mémoires d'images unitaires sont adaptées pour stocker la pluralité d'images unitaires, et la section de correction d'images est adaptée pour corriger chaque image de la pluralité d'images unitaires.

13. Appareil de diagnostic à ultrasons selon la revendication 9, adapté pour commander le facteur de pondération pour une image unitaire conformément au vecteur de mouvement.

# FIG.1

ULTRASONIC IMAGE CONSTRUCTING UNIT

# FIG.2

CONSTRUCTION OF B-mode IMAGE $f_n$ OF SUBJECT UNDER INSPECTION ~STEP1

$n = n+1$

$n = 1$ — YES

NO

STORING OF IMAGE $f_1$ IN IMAGE MEMORY #1

STEP2

READING OF IMAGE $f_{n-1}$ FROM IMAGE MEMORY #1 ~STEP3

DEFINITION OF REGION FOR DETECTING MOTION VECTOR ON IMAGE $f_{n-1}$ ~STEP4

DETECTION OF MOTION VECTOR (BETWEEN IMAGE $f_n$ AND IMAGE $f_{n-1}$) ~STEP5

TRANSFORMATION OF ADDED IMAGES ~STEP9

WEIGHTING PROCESS ~STEP10

IMAGE ADDING PROCESS ~STEP6

STORING OF ADDED IMAGE ~STEP7

DISPLAY OF ADDED IMAGE ~STEP8

$n = n+1$

$f_n$

23

22

21

FIG.3

$f_{n-1}$

24

23

26

22

25

EP 1 990 009 B1

# FIG.4

$$n=1 \rightarrow \boxed{f_n} \rightarrow \boxed{f_n \otimes f_{n-1}} \rightarrow \boxed{\text{TRANSFORMATION OF } \sum_{i=1}^{n-1} f_i} \rightarrow \boxed{\sum_{i=1}^{n} f_i = \alpha \sum_{i=1}^{n-1} f_i + \beta f_n}$$

41  42  43  44

$$n=n+1$$

Technology for displaying an added image using a small memory at a high speed

· FEATURE :
A detection of motion vector is carried out between two frames.
An added image is transformed based on the detection result of motion vector.
Setting of a weight factor.

· EFFECT :
Weighting is used to automatically remove artifact included in certain frames.

# FIG.5

| | |
|---|---|
| DEFINITION OF MEASUREMENT REGION | ～STEP1 |
| DEFINITION OF SEARCH REGION | ～STEP2 |
| LOW-PASS FILTER PROCESS OF MEASUREMENT REGION AND SEARCH REGION | ～STEP3 |
| DECIMATION OF MEASUREMENT REGION AND SEARCH REGION | ～STEP4 |
| DETECTION OF MOTION VECTOR | ～STEP5 |
| REDEFINITION OF SEARCH REGION | ～STEP6 |
| REDETECTION OF MOTION VECTOR | ～STEP7 |

FIG.6

$f_{n-1}'$   51

MEASUREMENT
REGION

$i \times j$

$f_n'$   52   53

$(i+20) \times (j+20)$

$f_{n-1}'$   54

MEASUREMENT
REGION

$i/2 \times j/2$

$f_n'$   55   56

$(i+20)/2 \times (j+20)/2$

52   53   57

V

56   55

# FIG.7

FIG.8

81

82

$\alpha = 0.85$

83

84

FIG.9

81

91

PRESS IN

α=0.85

83

PRESS IN

92

# FIG.10

# FIG.11

B-mode IMAGE MEMORY

```
REFRESHING IMAGE       ~17
MEMORY #1

IMAGE MEMORY #1        ~11
```

IMAGE
DATA

```
MEASUREMENT REGION     ~12
SETTING SECTION
```

10

```
MOTION DETECTOR
```

ULTRASONIC IMAGE
CONSTRUCTING UNIT

MOTION VECTOR

16

```
WEIGHT
PARAMETER UNIT
```

```
MOTION VECTOR     ~18
MEMORY
```

MOTION
VECTOR

13

```
ACCUMULATION
UNIT
```

19

```
IMAGE
SUBTRACTION UNIT
```

204

```
DEFORMATION
UNIT
```

14                              20

```
IMAGE MEMORY #2        REFRESHING IMAGE
                       MEMORY #2
```

CUMULATIVE
ADDED IMAGE

MEMORY FOR
ADDED IMAGE

```
DISPLAYING
SECTION          ~15
```

# FIG.12

```
                    IMAGE MEMORY #1        ~11
                           ↓
          IMAGE     MEASUREMENT REGION     ~12
          DATA      SETTING SECTION
                           ↓
                    MOTION DETECTOR  ←  ~10   ┌─────────────────┐
                                             │ ULTRASONIC IMAGE│
                    IMAGE                     │ CONSTRUCTING UNIT│
                    DATA     MOTION VECTOR    └─────────────────┘
                      ↓         ↓
            16~   WEIGHT          ←  DEFORMATION   ~204
                  PARAMETER UNIT     UNIT
                      ↓                  ↑
            13~   ACCUMULATION  →   UNIT IMAGE     ~203
                  UNIT              MEMORIES
                      ↓
            15~   DISPLAYING
                  SECTION
```

# FIG.13

UNIT IMAGE MEMORIES

```
┌───────────────────────────────────────────────────────────────────┐
│  UNIT      UNIT      UNIT      UNIT      UNIT                       │
│  IMAGE     IMAGE     IMAGE     IMAGE     IMAGE                      │
│    5         10        15        20        25                      │
│   Σ  f      Σ  f      Σ  f      Σ  f      Σ  f                      │
│   i=1 i     i=6 i     i=11 i    i=16 i    i=21 i                    │
└───────────────────────────────────────────────────────────────────┘
```

$$\sum_{i=1}^{5} f_i \qquad \sum_{i=6}^{10} f_i \qquad \sum_{i=11}^{15} f_i \qquad \sum_{i=16}^{20} f_i \qquad \sum_{i=21}^{25} f_i$$

```
1        5 6      10 11     15 16     20 21      25 26  FRAME
301  302                                               NUMBER
```

## FIG.14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002209898 A
- JP 3447150 B

- US 5495846 A

**Non-patent literature cited in the description**

- **K. ISHIHARA et al.** High-speed digital subtraction echography: Principle and preliminary application to arteriosclerosis, arrhythmia and blood flow visualization. *Ultrasonics Symposium,* 1990, 1473-1476